# EUROPEAN PATENT SPECIFICATION

(11) **EP 3 861 336 B1**
(45) Date of publication and mention of the grant of the patent: **24.01.2024**
(21) Application number: 19787329.2
(22) Date of filing: 27.09.2019
(51) Int. Cl.: G01N 31/22, G01N 21/64, G01N 21/77

(54) **METHOD FOR MEASURING CONCENTRATION OF POLYELECTROLYTE AND PHOSPHONATE BLENDS**
VERFAHREN ZUR MESSUNG DER KONZENTRATION VON POLYELEKTROLYTEN UND PHOSPHONATEN IN MISCHUNGEN
PROCÉDÉ DE MESURE DE LA CONCENTRATION DE POLYÉLECTROLYTES ET DE PHOSPHONATES DANS DES MÉLANGES

(30) Priority: 01.10.2018 FI 20185816
(43) Date of publication of application: 11.08.2021
(73) Proprietor: KEMIRA OYJ, 00180 Helsinki (FI)
(72) Inventor: PUUPPONEN, Salla, 02270 Espoo (FI); KRAPU, Sari, 02270 Espoo (FI)
(74) Representative: Berggren Oy
(86) International application number: PCT/FI2019/050694
(87) International publication number: WO 2020/070385

(56) References cited:
- WO-A1-2015/075299
- WO-A1-2015/075300
- WO-A1-2016/066885
- DE-A1-102008 006 610
- INNA V MELNYK ET AL: "Dy(III) sorption from water solutions by mesoporous silicas functionalized with phosphonic acid groups", JOURNAL OF POROUS MATERIALS, KLUWER ACADEMIC PUBLISHERS, BO, vol. 19, no. 5, 23 August 2011 (2011-08-23), pages 579-585, XP035114647, ISSN: 1573-4854, DOI: 10.1007/S10934-011-9508-3

## Description

### Field of invention

The present invention relates to a method for determining concentration of polyelectrolyte and phosphonate blends in a sample with time-resolved fluorescence.

### Background

Quantification of polyelectrolytes such as organic polymers is essential in many fields of industry, such as in water treatment, paper and oil industries. The organic polymers desired to be analyzed may be corrosion inhibitors, antiscalants or enhanced oil recovery (EOR) polymers.

Organic polymers can be measured in a rapid and easy manner by utilizing time-resolved fluorescence (TRF) of lanthanides(III). Europium(III) and terbium(III) are the most used lanthanide ions in TRF measurements, but also other lanthanides can be used. Organic polymers containing two or more chelating groups often increase the inherently weak TRF intensity of lanthanide(III) ions.

The chelating groups of the polymer may be e.g. carboxylates, sulfonates, carboxamides, phosphates, phosphonates or amines. The signal amplification depends on the polymer chemistry and concentration. Therefore, the signal amplification of polymer-lanthanide(III) chelates can be exploited for quantification of polymer samples, given that the chemistry (particularly that of chelating groups) of the sample does not substantially vary.

Often, polymer blends are used as products instead of single polymers. For instance, copolymer of sodium allyl sulfonate and maleate (SASMAC) scale inhibitors may be used together with polyacrylate type antiscalants as a blend in water treatment processes due to their different operation principles. If the fractions of the polymers in the blend change in use, reliable quantification has been challenging or even impossible, because the polymers give different TRF signals.

Therefore, there is need for a simple and reliable method for measuring total concentration of polyelectrolyte and phosphonate blends.

WO 2016/066885 discloses a method for quantifying phosphonates with low detection limit by admixing a sample including phosphonates with lanthanide(III) ions in a buffer solution wherein the buffer solution includes a buffering agent which has pKₐ value from 6 to 10. The buffering agent does not include more than one COOH groups but it includes one or more OH groups.

WO 2015/075299 discloses a method for determining samples including one or more organic polymers by mixing them with lanthanide(III) ions and detecting the signal of the lanthanide(III) ion chelated with the organic polymer.

DE 102008006610 discloses a method for detecting an analyte containing polyamino acid with a high sensitivity.

Melnyk et al., J. Porous Mater. (2012) 19:579-585 discloses Dy(III) sorption from water solutions by mesoporous silicas functionalised with phosphonic acid groups.

WO 2015/075300 discloses a method for examining samples including one or more organic polymers including two or more groups detected from carboxylates, sulfonates, phosphates, phosphonates, carboxamides and amines. The method includes two or more sub-methods wherein at least one of the sub-methods includes quantifying one organic polymer with the aid of lanthanide(III) ions.

### Summary of invention

An object of the present invention is to provide method for measuring total concentration of polyelectrolyte and phosphonate blends.

Another object of the present invention is to provide a simple and reliable method for measuring total concentration polyelectrolyte and phosphonate blends.

Chelation tendency and TRF signal amplification of polyelectrolyte or phosphonate -lanthanide mixtures depend often substantially on the charging (chelating groups) of the polymer.

In the present invention, the different TRF signals of distinct polyelectrolytes are standardized by suitable sample pre-treatment and/or modification of the measurement conditions.

It was found that the TRF signal intensity of lanthanide(III)-polyacrylic acid mixtures was substantially higher than those of lanthanide(III)-polyacrylates and lanthanide(III)-SASMAC mixtures.

It was also surprisingly found that the different signals of the polyelectrolyte and phosphonate could be standardized to the same level by acidification of the sample. Suitable acidification treatments change the TRF signal of the different polymers into the same level, enabling quantification of polyelectrolyte and phosphonate blends independent on the fractions of the polymers present in the sample.

By combining the total measurement of the present invention with suitable separation methods of the analytes or measurement methods specific for single polyelectrolyte or phosphonate in blend, the polyelectrolyte and phosphonate blend compositions can be completely solved.

In the method of the present invention aqueous sample solution is acidified to a pH of 6 or less, such as 4-6.

In one embodiment, when sample matrix chemistry varies, the pH level can be standardized by using buffer in the acidification reaction. Preferably, the buffer is non-chelating, zwitterionic Good's buffer, such as HEPES or tris-bis propane. The buffer is selected so that it works in the modified pH range.

After standardization procedures, all the analytes (polyelectrolytes and phosphonates) give similar TRF signal. The signal of the blend can be converted into ppm range by comparing the signal to calibration curve. The calibration standards should be treated in the same manner as the analytes. The polyelectrolytes contain two or more chelating groups selected from esters, ethers, hydroxyls, thiols, carboxylates, sulfonates, amides, phosphates, phosphonates and/or amines.

### Brief description of figures

Figure 1 illustrates time-resolved fluorescence signal of lanthanide(III)-polymer chelates.
Figure 2 illustrates time-resolved fluorescence signal of Europium with 0-10 ppm polymeric scale inhibitors.

### Detailed description

The present invention provides a method for determining total concentration of polyelectrolyte and phosphonate blends in a sample. More particularly the present invention provides a method for determining total concentration of polyelectrolyte and phosphonate in a sample comprising two or more polyelectrolyte or phosphonate, the method comprising
- optionally diluting and/or purifying the sample,
- acidifying the sample to pH less than 6,
- admixing the acidified sample with a reagent comprising lanthanide(III) ion,
- allowing the organic polymers in the sample to interact with the reagent comprising the lanthanide(III) ion,
- exciting the sample at an excitation wavelength and detecting a sample signal deriving from the lanthanide(III) ion at a signal wavelength by using time-resolved fluorescence, and
- determining the total concentration of polyelectrolyte and phosphonate in the sample by using the detected sample signal.

The pH of the sample is preferably adjusted to range 4-6.

In one embodiment the sample is acidified with an acid selected from a group consisting of HCl, HNO₃, H₂SO₄ or H₃PO₄, preferably HCl.

In a preferred embodiment additionally a buffer is admixed with the sample, preferably non-chelating buffer, more preferably zwitterionic Good's buffer, even more preferably HEPES or tris-bis propane.

In one embodiment concentration of the polyelectrolyte or phosphonate in the measurement mixture is in the range of 0.5 - 50 ppm, preferably 5-20 ppm.

In case the concentration of the polyelectrolyte and phosphonate in the sample is higher, the sample can be diluted.

In one embodiment concentration of the lanthanide(III) ion in the measurement mixture is in the range of 1-100µM), preferably 1-20µM.

By term "measurement mixture" is meant the admixture in the measurement.

The lanthanide(III) ion is selected from europium, terbium, samarium or dysprosium ions. The lanthanide (III) ion is preferably europium or terbium ions.

In a preferred embodiment the lanthanide(III) is a lanthanide(III) salt. The lanthanide(III) salt is selected from halogenides and oxyanions, such as nitrates, sulfates or carbonates, preferably from hydrated halogenides or nitrates, more preferably hydrated chloride.

The sample is optionally diluted to suitable aqueous solution e.g. deionized water or brine containing monovalent and/or divalent ions. Preferably, the dissolution brine does not contain any trivalent ions. Preferably the sample is an aqueous solution.

If the sample solution contains some interfering compounds such as trivalent metal cations or chelating agents that may affect TRF signal, suitable purification procedures may be applied prior to the dilution steps.

The sample is optionally purified by using a purification method selected from centrifugation, size exclusion chromatography, cleaning with solid-phase extraction (SPE) cartridges, dialysis techniques, extraction methods for removing hydrocarbons, filtration, microfiltration, ultrafiltration, nanofiltration, membrane centrifugation and any combinations thereof.

The analyte, polyelectrolyte or phosphonate, in the sample bears one or more groups that can hydrolyze and/or that the sample bears one or more groups that are capable of dissociating in aqueous solution to form either anion or cation groups. The analyte can be zwitterionic, i.e. contain both cationic and anionic groups. The charging of the group can depend on the environment pH (acidic or basic groups, such as carboxylic acids and amino groups). Therefore, the groups capable for dissociation can be neutral in certain pH (e.g. carboxylic acid group in acidic and amino groups in basic environment). The polyelectrolyte can be basic or acidic.

In one embodiment the polyelectrolyte contains one or more groups selected from, carboxylic acid/carboxylate, amide, phosphonate, amine, or any combination thereof.

In a preferred method the polyelectrolyte contain aromatic groups. The aromatic group(s) amplify the signal of lanthanide(III) ion.

In one embodiment the polyelectrolyte has molecular weight of at least 1000 g/mol.

In another embodiment the phosphonate has molecular weight of at least 100 g/mol.

To determine concentration of one polyelectrolyte or phosphonate in the blend said polyelectrolyte or phosphonate can be tagged.

In one embodiment at least one of the polyelectrolyte or phosphonate is tagged, preferably with fluorescent tag, such as sodium styrene sulfonate (NaSS), with different excitation and emission wavelengths as compared to the used lanthanide(III).

In said embodiment, after the determination of the total concentration of the polyelectrolyte or phosphonate polymers in the sample, the sample is
- exicated at excitation wavelength specific for the tag and detecting a sample signal deriving from the lanthanide(III) ion at a signal wavelength specific for the tag by using time-resolved fluorescence, and
- determining the concentration of the at least one tagged polyelectrolyte or phosphonate in the sample by using the detected sample signal.

Unknown concentration of the polyelectrolyte or phosphonate in the sample is determined by comparing the sample signal to calibration curve. The calibration curve is obtained from TRF measurement of calibration standard samples with varying polyelectrolyte or phosphonate concentrations. Same dilution and or purification steps and measurement parameters have to be used for both the sample and calibration samples.

The lanthanide(III) ion is excited at excitation wavelength and measured at emission wavelength and detected by using time-resolved fluorescence (TRF) . Any TRF reader can be employed. Excitation and emission wavelengths are selected so that the S/N is the best. Also the delay time can be optimized.

The excitation and emission wavelengths and the delay time are chosen based on the requirements of the lanthanide ion.

In an exemplary embodiment excitation wavelength and emission wavelength and delay time for Europium is 395 nm and 615 nm and 400 µs respectively.

The present invention further relates to use of the method of the present invention for determining total concentration of polyelectrolyte and/or phosphonate in a sample.

The sample can originate from water treatment, paper making processes, pharmaceutical industry, well drilling, mineral processing, enhanced oil recovery, an oilfield or an oil well or from an oil production process.

The present invention further relates a device comprising means for performing the method according to the present invention for determining total concentration of polyelectrolyte or phosphonate in a sample.

The examples are not intended to limit the scope of the invention but to describe embodiments of the invention.

### Examples

### Example 1. Standardization (modification) of the TRF signal of polyacrylic acids, polyacrylates, SASMAC and AA-AMPS copolymers or a polymer blend thereof (according to the invention)

The polymer or polymer blend is optionally diluted to suitable concentration range. Preferably, the active concentration of the sample and europium lanthanide are 0.5-50 ppm and µM, respectively. Optionally the polymer is diluted to suitable aqueous solution e.g. deionized water or brine containing monovalent and/or divalent ions. Preferably, the dissolution brine does not contain any trivalent ions. If the polymer solution contains some interfering compounds, suitable pretreatment procedures may be applied prior to the dilution steps. In the example, the polymers were diluted into a brine, which TDS varied between 20000 and 40 000 ppm. The brines contain alkaline and earth alkaline metals as chlorides or bicarbonates.

After the optional dilution of the polymer, the solution is acidified to a under 6.

If the sample matrix chemistry varies, the pH level can be standardized also by using buffer in the reaction. Preferably, the buffer is non-chelating, zwitterionic Good's buffer, such as HEPES or tris-bis propane. The buffer is selected so that it works in the selected, modified pH range, e.g. in pH of 4-6.

After the standardization (modification) procedures, all the analytes give similar TRF signal. The signal of the blend can be converted into ppm range by comparing the signal to the calibration curve. The calibration standards should be treated in the same manner as the analytes.

### Example 2. Binary mixture of polyacrylic acid and fluorescence tagged SASMAC (according to the invention)

The binary mixture composition of polyacrylic acid and fluorescence tagged SASMAC can be examined by first using the modification method presented in Example 1. In the modification, the high signal of polyacrylic acid is reduced into the same level as that of SASMAC.

After total measurement, tagged SASMAC polymer can be measured with fluorescence method specific for the tag. For instance, sodium styrene sulfonate (NaSS) tagged SASMAC polymer can be quantified by fluorescence measurement of the product. Tag specific excitation and emission wavelengths are used in the measurement. In the case of NaSS tag, excitation and emission wavelengths of 225 nm and 285 nm can be used. The calibration standards of NaSS tagged SASMAC are measured similarly as the product and the fluorescence signal are converted into concentration of the polymer.

Polyacrylic acid concentration is obtained by subtracting the SASMAC polymer concentration from the total signal.

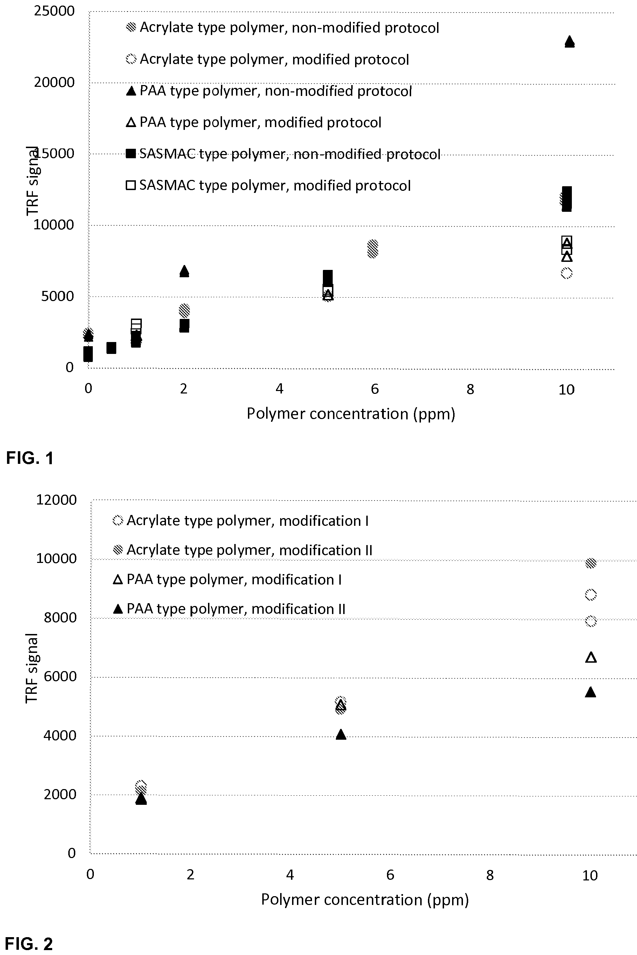

The signal is measured from non-modified polymers and from polymers modified with the method of the present invention. In non-modified protocol, the pH is adjusted to 6.8 using HEPES buffer. In modified protocol, the pH is ~5 in the reaction. No buffer is used in the modified protocol. As can be seen from Figure 1 the signals of the modified polymers are at same level compared to signals of non-modified polymers.

Table 1 presents comparison of the TRF signals of lanthanide-polymer chelates measured with non-modified protocol and modified protocol. In non-modified protocol, the pH is adjusted to 6.8 using HEPES buffer. In modified protocol (modification I), the pH is ~5 in the reaction. No buffer is used in the modified protocol. Max difference of the TRF signals - maximum difference of the polymer TRF signal as compared to other polymers measured (with same concentration range). It can be observed that the differences between the signals are -35-130% for non-modified protocol, whereas the differences in the TRF signals are -5-50% for modification (mostly, below 35%).

**Table 1.**

| Polymer | Concentratio n (ppm) | TRF signal | | Max difference of the TRF signals | |
|---|---|---|---|---|---|
| | | Non-modified protocol | Modified protocol | Non-modified protocol | Modifie d protocol |
| polyacrylic acid, pH of product 3.90 | 1 | 4520* | 2290 | 130 % | 20 % |
| | 5 | 12720* | 5170 | 100 % | 5% |
| | 10 | 23000 | 8370 | 100 % | 25 % |
| sodium polyacrylate , pH of product 6.66 | 1 | 3260* | 1890 | 70 % | 35 % |
| | 5 | 8380 | 5070 | 35 % | 5% |
| | 10 | 11880 | 6710 | 50 % | 20 % |
| SASMAC polymer | 1 | 1990 | 2870 | 55 % | 50 % |
| | 5 | 6380 | 5470 | 50 % | 10% |
| | 10 | 11960 | 8670 | 50 % | 30 % |

Figure 2 illustrates Europium time resolved fluorescence signal with 0-10 ppm polymeric scale inhibitors. Used polymers are polyacrylic acid (PAA type polymer), pH of product 3.90; and sodium polyacrylate (acrylate type polymer), pH of product 6.66. In modification I, the pH of the sample is adjusted to ~5. No buffer is used in the modified protocol. In modification II, the pH of the sample is adjusted to ~5 prior to measurement and HEPES buffer is used. The pH of the measurement solution is ~6. The concentration of Eu is 11.4 µM in all the tests.

## Claims

1. A method for determining total concentration of polyelectrolyte or phosphonate in a sample comprising two or more polyelectrolytes and/or phosphonates, the method comprising
- optionally diluting and/or purifying the sample,
- acidifying the sample to pH less than 6,
- admixing the acidified sample with a reagent comprising lanthanide(III) ion,
- allowing the polyelectrolyte or phosphonate in the sample to interact with the reagent comprising the lanthanide(III) ion,
- exciting the sample at an excitation wavelength and detecting a sample signal deriving from the lanthanide(III) ion at a signal wavelength by using time-resolved fluorescence, and
- determining the total concentration of the polyelectrolyte or phosphonate in the sample by using the detected sample signal.

2. The method according to claim 1, wherein additionally a buffer is admixed with the sample, preferably non-chelating buffer, more preferably zwitterionic Good's buffer, even more preferably HEPES or tris-bis propane.

3. Method according to any of claims 1-2, wherein the sample is acidified with an acid selected from a group consisting of HCl, HNO₃, H₂SO₄ or H₃PO₄, preferably HCl.

4. Method according to any of claims 1-3, wherein concentration of the lanthanide(III) ion in the measurement mixture is in the range of 1-100 µM, preferably 1-20 µM.

5. Method according to any one of claims 1-4, wherein concentration of the polyelectrolyte or phosphonate in the measurement mixture is in the range of 0.5-50 ppm, preferably 5-20 ppm.

6. Method according to any one of claims 1-5, wherein the lanthanide(III) ion is selected from europium, terbium, samarium or dysprosium ions, preferably europium or terbium ions.

7. Method according to any one of claims 1-6, wherein the lanthanide(III) is a lanthanide(III) salt, preferably halogenide or oxyanion, more preferably hydrated halogenides or nitrates, most preferably hydrated chloride.

8. Method according to any of claims 1-7, wherein the sample is purified by using a purification method selected from centrifugation, size exclusion chromatography, cleaning with solid-phase extraction (SPE) cartridges, dialysis techniques, extraction methods for removing hydrocarbons, filtration, microfiltration, ultrafiltration, nanofiltration, membrane centrifugation and any combinations thereof.

9. Method according to any of claims 1-8, wherein the polyelectrolyte contains one or more groups selected from, carboxylic acid/carboxylate, amide, phosphonate, amine, or any combination thereof.

10. Method according to any of claims 1-9, wherein at least one of the polyelectrolytes or phosphonates is tagged, preferably with fluorescent tag with different excitation and emission wavelengths as compared to the used lanthanide(III).

11. Method according to claim 10, wherein after the determination of the total concentration of the polyelectrolyte or phosphonate in the sample, the sample is
- excited at excitation wavelength specific for the tag and detecting a sample signal deriving from the lanthanide(III) ion at a signal wavelength specific for the tag by using time-resolved fluorescence, and
- determining the concentration of the at least one tagged polyelectrolyte or phosphonate in the sample by using the detected sample signal.

12. The method according to claim 1, wherein the sample originates from water treatment, paper making processes, pharmaceutical industry, well drilling, mineral processing, enhanced oil recovery, an oilfield or an oil well or from an oil production process.

## Patentansprüche

1. Verfahren zum Bestimmen der Gesamtkonzentration von Polyelektrolyten oder Phosphonaten in einer Probe, die zwei oder mehr Polyelektrolyte und/oder Phosphonate umfasst, wobei das Verfahren umfasst
- gegebenenfalls Verdünnen und/oder Reinigen der Probe,
- Ansäuern der Probe auf einen pH-Wert von weniger als 6,
- Mischen der angesäuerten Probe mit einem Reagens, das ein Lanthanid(III)-Ion umfasst,
- Zulassen, dass der Polyelektrolyt oder das Phosphonat in der Probe mit dem Reagens, das das Lanthanid(III)-Ion umfasst, wechselwirkt,
- Anregen der Probe mit einer Anregungswellenlänge und Erfassen eines Probensignals, das von dem Lanthanid(III)-Ion stammt, mit einer Signalwellenlänge unter Verwendung von zeitaufgelöster Fluoreszenz, und
- Bestimmen der Gesamtkonzentration des Polyelektrolyts oder Phosphonats in der Probe unter Verwendung des erfassten Probensignals.

2. Verfahren nach Anspruch 1, wobei zusätzlich ein Puffer in die Probe gemischt wird, vorzugsweise ein nicht chelatbildender Puffer, bevorzugter ein zwitterionischer Good-Puffer, noch bevorzugter HEPES oder Tris-bis-Propan.

3. Verfahren nach einem der Ansprüche 1-2, wobei die Probe mit einer Säure angesäuert wird, die aus einer Gruppe ausgewählt ist, die aus HCl, HNO₃, H₂SO₄ oder H₃PO₄ besteht, vorzugsweise HCl.

4. Verfahren nach einem der Ansprüche 1-3, wobei die Konzentration des Lanthanid(III)-Ions in dem Messgemisch im Bereich von 1-100 µM, vorzugsweise 1-20 µM, liegt.

5. Verfahren nach einem der Ansprüche 1-4, wobei die Konzentration des Polyelektrolyts oder Phosphonats in dem Messgemisch im Bereich von 0,5-50 ppm, vorzugsweise 5-20 ppm, liegt.

6. Verfahren nach einem der Ansprüche 1-5, wobei das Lanthanid(III)-Ion ausgewählt ist aus Europium-, Terbium-, Samarium- oder Dysprosiumionen, vorzugsweise Europium- oder Terbiumionen.

7. Verfahren nach einem der Ansprüche 1-6, wobei das Lanthanid(III) ein Lanthanid(III)-Salz ist, vorzugsweise Halogenid- oder Oxyanion, bevorzugter hydrierte Halogenide oder Nitrate, höchst bevorzugt hydriertes Chlorid.

8. Verfahren nach einem der Ansprüche 1-7, wobei die Probe unter Verwendung eines Reinigungsverfahrens, ausgewählt aus Zentrifugation, Größenausschlusschromatographie, Reinigung mit Festphasenextraktion(SPE)-Kartuschen, Dialyseverfahren, Extraktionsverfahren zum Entfernen von Kohlenwasserstoffen, Filtration, Mikrofiltration, Ultrafiltration, Nanofiltration, Membranzentrifugation und beliebigen Kombinationen davon, gereinigt wird.

9. Verfahren nach einem der Ansprüche 1-8, wobei der Polyelektrolyt eine oder mehrere Gruppen enthält, ausgewählt aus Carbonsäure/Carboxylat, Amid, Phosphonat, Amin oder einer beliebigen Kombination davon.

10. Verfahren nach einem der Ansprüche 1-9, wobei mindestens einer der Polyelektrolyte oder Phosphonate markiert ist, vorzugsweise mit einer Fluoreszenzmarkierung mit unterschiedlichen Anregungs- und Emissionswellenlängen im Vergleich zum verwendeten Lanthanid(III).

11. Verfahren nach Anspruch 10, wobei nach der Bestimmung der Gesamtkonzentration des Polyelektrolyts oder Phosphonats in der Probe die Probe
- mit einer für die Markierung spezifischen Anregungswellenlänge angeregt wird und ein Probensignal, das von dem Lanthanid(III)-Ion stammt, bei einer für die Markierung spezifischen Signalwellenlänge unter Verwendung zeitaufgelöster Fluoreszenz erfasst wird, und
- die Konzentration des mindestens einen markierten Polyelektrolyts oder Phosphonats in der Probe unter Verwendung des erfassten Probensignals bestimmt wird.

12. Verfahren nach Anspruch 1, wobei die Probe aus der Wasseraufbereitung, Papierherstellungsprozessen, der pharmazeutischen Industrie, Brunnenbohrungen, der Mineralverarbeitung, der sekundären Ölförderung, einem Ölfeld oder einer Ölquelle oder aus einem Ölproduktionsprozess stammt.

## Revendications

1. Procédé pour déterminer la concentration totale de polyélectrolyte ou de phosphonate dans un échantillon comprenant au moins deux polyélectrolytes et/ou phosphonates, le procédé comprenant les étapes consistant à
- éventuellement diluer et/ou purifier l'échantillon,
- acidifier l'échantillon à un pH inférieur à 6,
- mélanger l'échantillon acidifié à un réactif comprenant l'ion lanthanide(lll),
- permettre au polyélectrolyte ou au phosphonate dans l'échantillon d'interagir avec le réactif comprenant l'ion lanthanide(lll),
- exciter l'échantillon à une longueur d'onde d'excitation et détecter un signal d'échantillon dérivé de l'ion lanthanide(lll) à une longueur d'onde de signal en utilisant une fluorescence résolue en temps, et
- déterminer la concentration totale du polyélectrolyte ou du phosphonate dans l'échantillon à l'aide du signal d'échantillon détecté.

2. Procédé selon la revendication 1, dans lequel un tampon est en outre mélangé à l'échantillon, de préférence un tampon non chélatant, plus préférablement un tampon de Good zwitterionique, encore plus préférablement HEPES ou tris-bis propane.

3. Procédé selon l'une quelconque des revendications 1 ou 2, dans lequel l'échantillon est acidifié avec un acide choisi dans un groupe constitué de HCl, HNO₃, H₂SO₄ ou H₃PO₄, de préférence HCl.

4. Procédé selon l'une quelconque des revendications 1 à 3, dans lequel la concentration de l'ion lanthanide(lll) dans le mélange de mesure est comprise dans la plage de 1 à 100 µM, de préférence de 1 à 20 µM.

5. Procédé selon l'une quelconque des revendications 1 à 4, dans lequel la concentration du polyélectrolyte ou du phosphonate dans le mélange de mesure est comprise dans la plage de 0,5 à 50 ppm, de préférence de 5 à 20 ppm.

6. Procédé selon l'une quelconque des revendications 1 à 5, dans lequel l'ion lanthanide(lll) est choisi parmi les ions europium, terbium, samarium ou dysprosium, de préférence les ions europium ou terbium.

7. Procédé selon l'une quelconque des revendications 1 à 6, dans lequel le lanthanide(lll) est un sel de lanthanide(lll), de préférence un halogénure ou un oxyanion, plus préférablement des halogénures ou des nitrates hydratés, de manière préférée entre toutes un chlorure hydraté.

8. Procédé selon l'une quelconque des revendications 1 à 7, dans lequel l'échantillon est purifié en utilisant un procédé de purification choisi parmi la centrifugation, la chromatographie d'exclusion stérique, le nettoyage avec des cartouches d'extraction en phase solide (SPE), les techniques de dialyse, les procédés d'extraction pour éliminer les hydrocarbures, la filtration, la microfiltration, l'ultrafiltration, la nanofiltration, la centrifugation sur membrane et toutes combinaisons associées.

9. Procédé selon l'une quelconque des revendications 1 à 8, dans lequel le polyélectrolyte contient un ou plusieurs groupes choisis parmi un acide carboxylique/carboxylate, un amide, un phosphonate, une amine ou toute combinaison associée.

10. Procédé selon l'une quelconque des revendications 1 à 9, dans lequel au moins l'un des polyélectrolytes ou phosphonates est marqué, de préférence avec un marqueur fluorescent ayant des longueurs d'onde d'excitation et d'émission différentes par rapport au lanthanide(lll) utilisé.

11. Procédé selon la revendication 10, dans lequel, après la détermination de la concentration totale du polyélectrolyte ou du phosphonate dans l'échantillon, l'échantillon est
- excité à une longueur d'onde d'excitation spécifique à l'étiquette et le procédé comprenant la détection d'un signal d'échantillon dérivé de l'ion lanthanide(lll) à une longueur d'onde de signal spécifique pour l'étiquette en utilisant une fluorescence résolue en temps, et
- la détermination de la concentration du ou des polyélectrolytes ou phosphonates marqués dans l'échantillon à l'aide du signal d'échantillon détecté.

12. Procédé selon la revendication 1, dans lequel l'échantillon provient du traitement de l'eau, des procédés de fabrication du papier, de l'industrie pharmaceutique, du forage de puits, du traitement des minéraux, de la récupération assistée du pétrole, d'un champ pétrolifère ou d'un puits de pétrole ou d'un procédé de production pétrolière.
